(19) **Europäisches Patentamt / European Patent Office / Office européen des brevets**

(11) **EP 4 534 017 B1**

(12) **EUROPEAN PATENT SPECIFICATION**

(45) Date of publication and mention
of the grant of the patent:
**04.02.2026 Bulletin 2026/06**

(21) Application number: **22943480.8**

(22) Date of filing: **26.10.2022**

(51) International Patent Classification (IPC):
*A61B 8/02* *(2006.01)*

(52) Cooperative Patent Classification (CPC):
**A61B 8/04; A61B 8/488; A61B 8/5223;** A61B 8/02;
A61B 8/06; A61B 8/0891

(86) International application number:
**PCT/CN2022/127744**

(87) International publication number:
**WO 2023/226293 (30.11.2023 Gazette 2023/48)**

(54) **DOPPLER ULTRASOUND-BASED CONTINUOUS BLOOD PRESSURE MEASURING APPARATUS AND ELECTRONIC DEVICE**

DOPPLER-ULTRASCHALLBASIERTE KONTINUIERLICHE BLUTDRUCKMESSVORRICHTUNG UND ELEKTRONISCHE VORRICHTUNG

APPAREIL DE MESURE CONTINUE DE PRESSION ARTÉRIELLE À BASE D'ULTRASONS DOPPLER ET DISPOSITIF ÉLECTRONIQUE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **24.05.2022 CN 202210568275**

(43) Date of publication of application:
**09.04.2025 Bulletin 2025/15**

(73) Proprietor: **Suzhou Sensus Medical Technology Co., Ltd.**
**Suzhou, Jiangsu 215200 (CN)**

(72) Inventors:
• **DING, Yan**
**Suzhou, Jiangsu 215200 (CN)**
• **SUN, Daniel**
**Suzhou, Jiangsu 215200 (CN)**

(74) Representative: **Lewis Silkin LLP**
**Arbor**
**255 Blackfriars Road**
**London SE1 9AX (GB)**

(56) References cited:
WO-A1-2020/246258      WO-A1-2020/246258
CN-A- 104 883 967      CN-A- 106 413 528
CN-A- 109 044 302      CN-A- 111 631 699
CN-A- 114 343 713      CN-A- 114 652 351
CN-B- 103 494 615      US-A1- 2014 180 114
US-A1- 2015 230 774      US-A1- 2018 199 834

## Description

CROSS REFERENCE TO RELATED APPLICATIONS

The present application claims priority to Chinese patent application No.202210568275.X, filed on May 24, 2022, entitled "CONTINUOUS BLOOD PRESSURE MEASURING METHOD AND APPARATUS BASED ON ULTRASONIC DOPPLER, AND ELECTRONIC DEVICE".

**TECHNICAL FIELD**

Embodiments of the present application relates to the technical field of continuous blood pressure measurement, and in particular, to a continuous blood pressure measuring apparatus based on ultrasonic Doppler, and electronic device.

**BACKGROUND**

In the conventional cuff blood pressure measurement, a single blood pressure measurement is often performed after using non-invasive inflation to block blood flow. Continuous blood pressure measurement is often required under the case of clinical monitoring or that there is a special need to observe continuous changes of the blood pressure. Therefore, the discontinuous conventional cuff blood pressure measurement is not applicable in this case. Currently, relevant continuous blood pressure measuring methods in clinical practice include invasive and non-invasive methods, in which the invasive method requires direct acquisition of the pressure in the blood vessels through puncture, and thus may easily cause discomfort to the user to be measured. Obviously, the non-invasive method has incomparable advantages over the invasive method for measuring blood pressure and the conventional cuff blood pressure measurement.

Continuous non-invasive measurement refers to a non-invasive and continuous measurement of blood pressure within a certain cycle of time, which can detect the blood pressure per beat and a continuous arterial pressure waveform to provide a more adequate basis for clinical diagnosis and treatment. The non-invasive and continuous method in the relevant technology is mainly obtained through the pulse wave analysis method. However, the pulse wave can also be interfered with by muscle motion, muscle elasticity feedback, etc., which causes the change of the pulse wave waveform, thereby reducing the accuracy of blood pressure measurement.

Specifically, the pulse wave mainly includes an envelope pressure pulse wave and a photoelectric volumetric pulse wave. For envelope pressure pulse wave analysis, it is necessary to pressurize a pressure detection part during measurement, one specific method for which is to set pressure measurement points at a plurality of parts, pressurize them in turns and periodically, and switch to another pressure measurement point for pressurization at every interval. However, after applying air pressure to the measurement part, the pressure waveform is still deformed due to the movement, motion or muscle contraction of the pressurized part, thereby causing motion noise. In addition, after the pressure compresses the blood vessel, the resistance of the blood may be interfered, which is also one of the factors affecting the accuracy of the measurement. At the same time, this method does not reduce the discomfort caused by the airbag restraining the human body during the blood pressure measurement process. For the photoelectric volumetric pulse wave analysis, the continuous blood pressure measurement using the photoelectric volumetric pulse wave can also be interfered by the motion or the surrounding light environment, thereby introducing measurement errors and reducing the accuracy of blood pressure measurement.

It can be seen that in the non-invasive continuous blood pressure measurement technology, further optimizing the experience of a user to be measured and improving the accuracy of blood pressure measurement still need to be solved.

US2015/230774A1 discloses a system and method for non-invasive, continuous, real time monitoring of a patient's arterial blood pressure.

SUMMARY OF THE DISCLOSURE.

The invention is set out in the appended set of claims.

In view of this, an embodiment of the present application provides a continuous blood pressure measuring method and apparatus based on ultrasonic Doppler, and electronic device to solve at least one problem existing in the background.

In a first aspect, an embodiment of the present application provides a continuous blood pressure measuring apparatus based on ultrasonic Doppler, including:

an acquisition module, configured to continuously acquire applied pressures during a pressurization operation performed from the beginning to the end on an artery to be measured, and continuously acquire Doppler blood flow signals of the artery to be measured, where the Doppler blood flow signals acquired during the pressurization operation are first Doppler blood flow signals, and the Doppler blood flow signals acquired after the pressurization operation are second Doppler blood flow signals;

a determination module, configured to obtain a maximum blood flow intensity of each of first pulse cycles according to the first Doppler blood flow signals, and determine a standard blood pressure from all of the acquired pressures according to all of the maximum blood flow intensities and changes thereof; and

a processing module, configured to perform spectrum envelope processing on the second Doppler blood flow signals to determine an initial blood flow parameter and a current blood flow parameter, and correct the standard blood pressure according to the initial blood flow parameter and the current blood flow parameter to obtain a current blood pressure.

In a second aspect, an embodiment of the present application provides a continuous blood pressure measuring apparatus based on ultrasonic Doppler, including:

a pressurizing portion, configured to apply pressure to an artery to be measured;

an ultrasonic Doppler sensor, configured to collect Doppler blood flow signals of the artery to be measured;

a blood pressure measuring instrument, connected to both of the ultrasonic Doppler sensor and the pressurizing portion and configured to:

continuously acquire applied pressures during a pressurization operation performed from the beginning to the end on the artery to be measured,

continuously acquire Doppler blood flow signals of the artery to be measured, wherein the Doppler blood flow signals acquired during the pressurization operation are first Doppler blood flow signals, and the Doppler blood flow signals acquired after the pressurization operation are second Doppler blood flow signals;

obtain a maximum blood flow intensity of each of first pulse cycles according to the first Doppler blood flow signals, and determine a standard blood pressure from all of the acquired pressures according to all of the maximum blood flow intensities and changes thereof; and

calculate an initial blood flow parameter and a current blood flow parameter by performing spectrum envelope processing on the second Doppler blood flow signals, and obtain a current blood pressure by correcting the standard blood pressure according to the initial blood flow parameter and the current blood flow parameter;

the specific step of obtaining a maximum blood flow intensity of each of first pulse cycles according to the first Doppler blood flow signals includes:

performing spectrum envelope processing on the first Doppler blood flow signals to obtain a first spectrum envelope curve;

sequentially identifying each of the first pulse cycles of the first spectrum envelope curve to obtain corresponding first pulse cycle curves; and

extracting a first maximum blood flow velocity from each of the first pulse cycle curves, where the first maximum blood flow velocity represents the maximum blood flow intensity,

the specific step of determining a standard blood pressure from all of the acquired pressures according to all of the maximum blood flow intensities and changes thereof includes:

if the acquired first maximum blood flow velocity is less than a set blood flow velocity threshold, determining a pressure corresponding to the acquired first maximum blood flow velocity as a systolic blood pressure $SBP_0$;

if a change of the acquired first maximum blood flow velocity compared with a first maximum blood flow velocity in a previous first pulse cycle meets a set constraint condition, determining a pressure corresponding to the acquired first maximum blood flow velocity as a diastolic blood pressure $DBP_0$; where the constraint condition includes a plurality of stored first maximum blood flow velocity data are searched, a pressure when the first maximum blood flow velocity begins to change is a diastolic blood pressure $DBP_0$, and "the first maximum blood flow velocity begins to change" is defined that an absolute value of a change between the current first maximum blood flow velocity and a first maximum blood flow velocity in a previous adjacent pulse cycle is 5%-10% ;

the specific step of calculating an initial blood flow parameter and a current blood flow parameter by performing spectrum envelope processing on the second Doppler blood flow signals includes:

performing the spectrum envelope processing on the second Doppler blood flow signals to obtain a second spectrum envelope curve;

sequentially identifying each of second pulse cycles of the second spectrum envelope curve to obtain corresponding second pulse cycle curves; and

extracting an initial blood flow parameter and a current blood flow parameter from initial and final second pulse cycle curves, where the blood flow parameter includes a maximum blood flow velocity, a velocity time integral, and a heart rate;

the specific step of obtaining a current blood pressure by correcting the standard blood pressure according to the initial blood flow parameter and the current blood flow parameter includes:

obtaining the current systolic blood pressure $SBP_n$ by correcting the systolic blood pressure $SBP_0$ in the standard blood pressure according to an initial value and a current value of the maximum blood flow velocity and an initial value and a current value of the velocity time integral;

obtaining the current diastolic blood pressure $DBP_n$ by correcting the diastolic blood pressure $DBP_0$ in the standard blood pressure according to the initial value and the current value of the maximum blood flow velocity and an initial value and a current value of the heart rate;

where the current systolic blood pressure $SBP_n$ is obtained according to a mathematical Equation:

$$SBP_n = SBP_0 + (b \cdot (Vti_n - Vti_0)/Vti_0 + a \cdot (Vmax_n - Vmax_0) / Vmax_0) \cdot SBP_0,$$

and

the current diastolic blood pressure $DBP_n$ is obtained according to a mathematical Equation:

$$DBP_n = DBP_0 + (x \cdot (Vmax_n - Vmax_0)/Vmax_0 + y \cdot (Hr_n - Hr_0)/Hr_0) \cdot DBP_0,$$

in the Equations, a and b respectively represent correction coefficients of relative errors of the maximum blood flow velocity and the velocity time integral in the solution of the current systolic blood pressure $SBP_n$, x and y respectively represent correction coefficients of relative errors of the maximum blood flow velocity and the heart rate in the solution of the current diastolic blood pressure $DBP_n$, $SBP_0$ and $DBP_0$ respectively represent the systolic blood pressure and the diastolic blood pressure in the standard blood pressure, and $SBP_n$ and $DBP_n$ respectively represent the systolic blood pressure and the diastolic blood pressure in the current blood pressure, and where the correction coefficients are constant.

In a third aspect, an embodiment of the present application provides an electronic device, including:

a processor;

a memory configured to store computer executable instructions;

wherein the processor is configured to execute the computer executable instructions to implement a continuous blood pressure measuring method based on ultrasonic Doppler, the continuous blood pressure measuring method including the following steps:

continuously acquiring applied pressures during a pressurization operation performed from the beginning to the end on the artery to be measured,

continuously acquiring Doppler blood flow signals of the artery to be measured, where the Doppler blood flow signals acquired during the pressurization operation are first Doppler blood flow signals, and the Doppler blood flow signals acquired after the pressurization operation are second Doppler blood flow signals;

obtaining a maximum blood flow intensity of each of first pulse cycles according to the first Doppler blood flow signals, and determining a standard blood pressure from all of the acquired pressures according to all of the maximum blood flow intensities and changes thereof; and

calculating an initial blood flow parameter and a current blood flow parameter by performing spectrum envelope processing on the second Doppler blood flow signals, and obtaining a current blood pressure by correcting the standard blood pressure according to the initial blood flow parameter and the current blood flow parameter,

where the specific step of obtaining a maximum blood flow intensity of each of first pulse cycles according to the first Doppler blood flow signals includes:

performing spectrum envelope processing on the first Doppler blood flow signals to obtain a first spectrum envelope curve;

sequentially identifying each of the first pulse cycles of the first spectrum envelope curve to obtain corresponding first pulse cycle curves; and

extracting a first maximum blood flow velocity from each of the first pulse cycle curves, wherein the first maximum blood flow velocity represents the maximum blood flow intensity,

where the specific step of determining a standard blood pressure from all of the acquired pressures according to all of the maximum blood flow intensities and changes thereof comprises:

if the acquired first maximum blood flow velocity is less than a set blood flow velocity threshold, determining a pressure corresponding to the acquired first maximum blood flow velocity as a systolic blood pressure $SBP_0$;

if a change of the acquired first maximum blood flow velocity compared with a first maximum blood flow velocity in a previous first pulse cycle meets a set constraint condition, determining a pressure corresponding to the acquired first maximum blood flow velocity as a diastolic blood pressure $DBP_0$, where the constraint condition includes a plurality of stored first maximum blood flow velocity data are searched, a pressure when the first maximum blood flow velocity begins to change is a diastolic blood pressure $DBP_0$, and "the first maximum blood flow velocity begins to change" is defined that an absolute value of a change between a current first maximum blood flow velocity and a first maximum blood flow velocity in a previous adjacent pulse cycle is 5%-10% ,

where the specific step of calculating an initial blood flow parameter and a current blood flow parameter by performing spectrum envelope processing on the second Doppler blood flow signals includes:

performing the spectrum envelope processing on the second Doppler blood flow signals to obtain a second spectrum envelope curve;

sequentially identifying each of second pulse cycles of the second spectrum envelope curve to obtain corresponding second pulse cycle curves; and

extracting an initial blood flow parameter and a current blood flow parameter from initial and final second pulse cycle curves, where the blood flow parameter comprises a maximum blood flow velocity, a velocity time integral, and a heart rate;

where the specific step of obtaining a current blood pressure by correcting the standard blood pressure according to the initial blood flow parameter and the current blood flow parameter includes:

obtaining a current systolic blood pressure $SBP_n$ by correcting the systolic blood pressure $SBP_0$ in the standard blood pressure according to an initial value and a current value of the maximum blood flow velocity and an initial value and a current value of the velocity time integral;

obtaining a current diastolic blood pressure $DBP_n$ by correcting the diastolic blood pressure $DBP_0$ in the standard blood pressure according to the initial value and the current value of the maximum blood flow velocity and an initial value and a current value of the heart rate,

where the current systolic blood pressure $SBP_n$ is obtained according to a mathematical Equation:

$$SBP_n = SBP_0 + (b \cdot (Vti_n - Vti_0)/Vti_0 + a \cdot (Vmax_n - Vmax_0)/Vmax_0) \cdot SBP_0,$$

and

the current diastolic blood pressure $DBP_n$ is obtained according to a mathematical Equation:

$$DBP_n = DBP_0 + (x \cdot (Vmax_n - Vmax_0)/Vmax_0 + y \cdot (Hr_n - Hr_0)/Hr_0) \cdot DBP_0,$$

in the Equations, a and b respectively represent correction coefficients of relative errors of the maximum blood flow velocity and the velocity time integral in the solution of the current systolic blood pressure $SBP_n$, x and y respectively represent correction coefficients of relative errors of the maximum blood flow velocity and the heart rate in the solution of the current diastolic blood pressure $DBP_n$, $SBP_0$ and $DBP_0$ respectively represent the systolic blood pressure and the diastolic blood pressure in the standard blood pressure, and $SBP_n$ and $DBP_n$ respectively represent the systolic blood pressure and the diastolic blood pressure in the current blood pressure, and where the correction coefficients are constant.

The beneficial effects brought by the technical solutions provided by the embodiments of the present application include:

By adopting ultrasonic Doppler blood flow measurement technology, except for compressing the artery to be measured during a single measurement of standard blood pressure, the subsequent Doppler blood flow signal acquisition does not require compression of the artery to be measured. Compared with the related technology that still needs to compress the artery to be measured during non-invasive continuous blood pressure measurement, it greatly reduces the compression of the arterial blood vessels, improves the experience of the user to be measured in continuous blood pressure measurement, and does not cause deformation of the pulse wave due to the interference of the pressure point on the blood flow, effectively improving the true accuracy of the previous sampling data, and thus improving the accuracy of the results in the non-invasive continuous measurement.

Additional aspects and advantages of the present application will be given in part in the description below, and in part will become apparent from the description below, or will be learned through the practice of the present application.

BRIEF DESCRIPTION OF DRAWINGS

The drawings described herein are used to provide a further understanding of the present application and constitute a part of the present application. The illustrative embodiments of the present application and the description thereof are used to explain the present application and do not constitute an improper limitation on the present application. In the drawings:

FIG. 1 is a schematic flow diagram of a continuous blood pressure measuring method based on ultrasonic Doppler provided in an embodiment of the present application;

FIG. 2 is a schematic diagram of the distribution of a continuous blood pressure measuring apparatus based on ultrasonic Doppler during measurement provided in an embodiment of the present application;

FIG. 3 is a schematic flow diagram of measuring a standard blood pressure for a single time in an embodiment of the present application;

FIG. 4 is a graph showing the curve relationship between the maximum current speed and the pressure in a method for measuring and calculating standard blood pressure in an embodiment of the present application;

FIG. 5 is a specific flow diagram of generating a spectrum envelope curve in an embodiment of the present application;

FIG. 6 is a schematic diagram of blood flow parameters on a spectrum envelope curve in an embodiment of the present application;

FIG. 7 is a schematic diagram of overlapping and fitting five initial pulse cycle curves on a spectrum envelope curve in an embodiment of the present application;

FIG. 8 is a schematic structural diagram of an electronic device provided in an embodiment of the present application; and

FIG. 9 is a schematic structural diagram of a continuous blood pressure measuring apparatus based on ultrasonic Doppler provided in an embodiment of the present application.

DETAILED DESCRIPTION

To make the technical solutions and beneficial effects of the present application more obvious and easy to understand, the detailed description is given below by enumerating specific embodiments. The accompanying drawings are not necessarily drawn to scale, and local features may be enlarged or reduced to show details of the local features more clearly. Unless defined otherwise, technical and scientific terms used herein have the same meaning as those in the technical field to which the present application belongs.

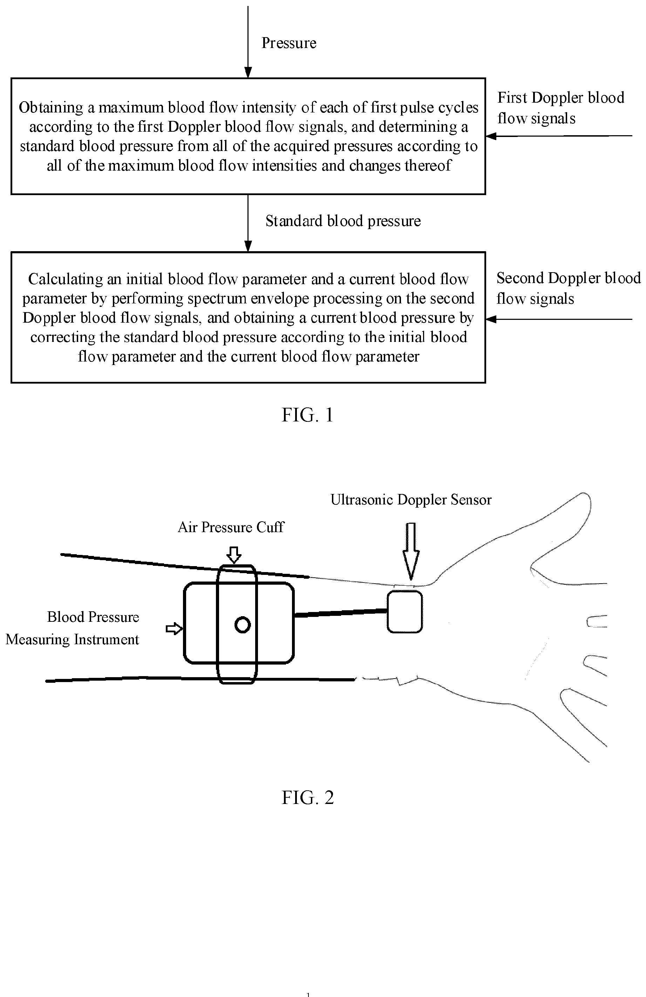

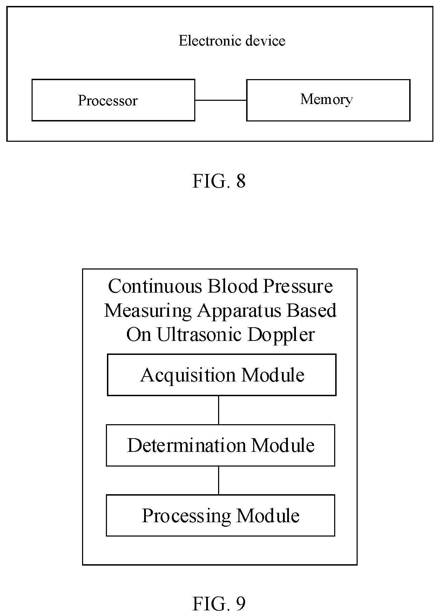

An embodiment of the present application provides a continuous blood pressure measuring apparatus based on ultrasonic Doppler. As shown in FIG. 9, the apparatus includes an acquisition module, a determination module, and a processing module. The apparatus is configured to perform a continuous blood pressure measuring method based on ultrasonic Doppler according to an embodiment of the present application. Specific process steps of the continuous blood pressure measuring method based on ultrasonic Doppler can refer to FIG. 1.

In conjunction with FIG. 1 and FIG. 9, in the continuous blood pressure measuring apparatus based on ultrasonic Doppler provided in the embodiment of the present application, the acquisition module is configured to continuously acquire an applied pressure during a pressurization operation performed from the beginning to the end on an artery to be measured, and continuously acquire Doppler blood flow signals of the artery to be measured, where the Doppler blood flow signals acquired during the pressurization operation are first Doppler blood flow signals, and the Doppler blood flow signals acquired after the pressurization operation are second Doppler blood flow signals; the determination module is configured to obtain a maximum blood flow intensity of each of first pulse cycles according to the first Doppler blood flow signals, and determine a standard blood pressure from all of the acquired pressures according to all of the maximum blood flow intensities and changes thereof; and the processing module is configured to perform spectrum envelope processing on the second Doppler blood flow signals to determine an initial blood flow parameter and a current blood flow parameter, and correct the standard blood pressure according to the initial blood flow parameter and the current blood flow parameter to obtain a current blood pressure.

In an embodiment of the present application, the continuous blood pressure measuring method is divided into two parts, one part is to obtain the standard blood pressure by a single measurement, and the other part is to obtain the current blood pressure by continuous measurement. In the pressurization operation, the standard blood pressure of the artery to be measured is obtained in a single measurement according to a relationship between the applied pressure and the maximum blood flow intensity in the acquired Doppler blood flow signals. Then, the Doppler blood flow signals of the artery to be measured are continuously acquired in a continuous and non-invasive method without pressurization, and the spectrum envelope processing is performed on the continuously acquired Doppler blood flow signals to calculate the current value and initial value of the blood flow parameter, so as to correct the standard blood pressure by obtaining the current value and initial value of the blood flow parameter to obtain the current blood pressure, thereby achieving the purpose of non-invasive continuous blood pressure measurement.

During a single measurement of the standard blood pressure, pressures are applied to the artery to be measured and the applied pressures are recorded. The pressures are measured from the beginning to the end of the pressurization. During this process, the first Doppler blood flow signals of the artery to be measured are continuously acquired. The maximum blood flow intensity of each of the first pulse cycles can be obtained according to the first Doppler blood flow signals, and then the standard blood pressure is determined from all of the acquired pressures according to all of the maximum blood flow intensities and changes thereof.

In the process of continuous blood pressure measurement, the Doppler blood flow signals, on which envelope processing is performed, are the second Doppler blood flow signals, which are acquired after the pressurization operation. The spectrum envelope processing is performed on the continuously acquired second Doppler blood flow signals to calculate the initial blood flow parameter and the current blood flow parameter, and the standard blood pressure is corrected to obtain the current blood pressure according to the initial blood flow parameter and the current blood flow parameter.

It should be noted that if the artery to be measured is pressurized in the form of inflation, then after the pressurization is ended, the Doppler blood flow signals acquired after the air is completely deflated are the second Doppler blood flow signals mentioned in the embodiment of the present application.

In the present embodiment, when the Doppler blood flow signal is needed to be acquired or collected, an ultrasonic Doppler sensor needs to be attached to the surface of the skin for collection, so that errors of the pulse waveform distortion caused after the compression is performed on the artery will be not generated. In addition to the blood pressure measurement on an arm as the artery to be measured, the continuous blood pressure measurement can also be applied for blood flow of other arteries, such as the carotid artery, the femoral artery, etc., in the embodiment of the present application. If the pressure sensor needs to have a corresponding pressure on the artery to sense and measure the pulse during the measurement, since a certain pressure is applied locally to the artery, the blood flow at the pressurized point will change, and the obtained pulse wave will also be deformed, which may cause errors.

As shown in FIG. 2, an ultrasonic Doppler sensor is attached to the artery to be measured, and an air pressure cuff is sleeved on the arm to pressurize the artery to be measured to block the blood flow by inflation. By connecting with the ultrasonic Doppler sensor and the air pressure cuff, the blood pressure measuring apparatus acquires the Doppler blood flow signals and pressures for single standard blood pressure measurement and non-invasive continuous blood pressure measurement.

As an optional embodiment of the present application, the determination module configured to determine the maximum blood flow intensity of each of first pulse cycles according to the first Doppler blood flow signals includes a determination module configured to execute:

performing spectrum envelope processing on the first Doppler blood flow signals to obtain a first spectrum envelope curve;
sequentially identifying each of the first pulse cycles of the first spectrum envelope curve to obtain corresponding first pulse cycle curves; and
extracting a first maximum blood flow velocity from each of the first pulse cycle curves, where the first maximum blood flow velocity represents the maximum blood flow intensity.

Further, the apparatus also includes a pressurization portion, which is configured to apply pressures to the artery to be measured, and is specifically configured to, during the pressurization operation performed from the beginning to the end on the artery to be measured, end the pressurization operation if the acquired first maximum blood flow velocity is less than a set blood flow velocity threshold, otherwise, continue the pressurization operation.

Further, the specific step of determining a standard blood pressure from all of the acquired pressures according to all of the maximum blood flow intensities and changes thereof includes:

if the acquired first maximum blood flow velocity is less than the set blood flow velocity threshold, determining a pressure corresponding to the acquired first maximum blood flow velocity as a systolic blood pressure $SBP_0$;

if the acquired first maximum blood flow velocity compared to a change of the first maximum blood flow velocity in a previous first pulse cycle meets a set constraint condition, determining a pressure corresponding to the obtained first maximum blood flow velocity as a diastolic blood pressure $DBP_0$. The constraint condition includes a plurality of stored first maximum blood flow velocity data are searched, the pressure when the first maximum blood flow velocity begins to change is the diastolic blood pressure $DBP_0$, and "the first maximum blood flow velocity begins to change" is defined that an absolute value of the change between the current first maximum blood flow velocity and a first maximum blood flow velocity in a previous adjacent pulse cycle is 5%-10%.

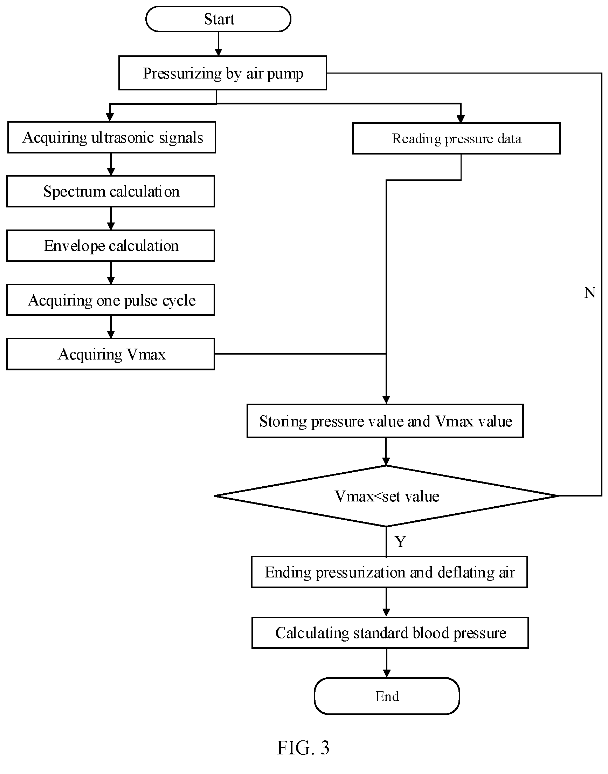

As shown in FIG. 3, an air pump in an air pressure cuff is controlled to pressurize, on the one hand, pressure data is read and stored, and on the other hand, ultrasonic signals are acquired periodically and synchronously, that is, the Doppler blood flow signals are acquired. Spectrum calculation and envelope calculation also need to be sequentially performed on the ultrasonic signals acquired in each cycle to acquire a pulse cycle, further the maximum blood flow velocity Vmax is acquired and stored. If the maximum blood flow velocity Vmax acquired in a pulse cycle is less than the set blood flow velocity threshold, that is, a set value, the pressurization is ended and the air is deflated for calculating the standard blood pressure, otherwise, the air pump continues to pressurize.

The set blood flow velocity threshold can be set to be between 0.2m/s and 0.4m/s according to an experience value, for example, 0.3m/s is specifically selected. The set blood flow velocity threshold can also be set to be 10% to 30% of the maximum value of the maximum blood flow velocities Vmax recorded during the measurement, for example, 20% is specifically selected.

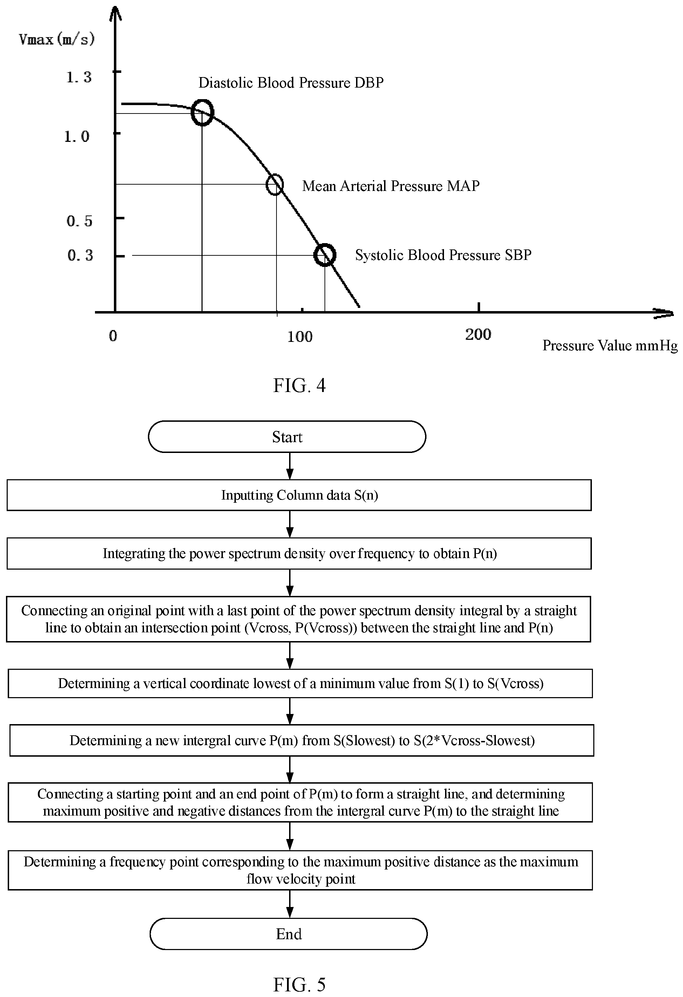

As shown in FIG. 4, in the measurement and calculation method for the standard blood pressure, when it is determined that the maximum blood flow velocity Vmax is less than the set blood flow velocity threshold, the pressure value at this time is the systolic blood pressure $SBP_0$. The plurality of stored maximum blood flow velocity Vmax data are searched, and the pressure when the maximum blood flow velocity Vmax value begins to change is the diastolic blood pressure $DBP_0$. Specifically, "when the maximum blood flow velocity Vmax value begins to change" is defined that an absolute value of the change between the current maximum blood flow velocity Vmax and the maximum blood flow velocity Vmax in the previous adjacent pulse cycle is 5%-10%, for example, 7% is specifically selected.

The mean arterial pressure $MAP=SBP-2/3 \cdot (SBP - DBP)=1/3 \cdot SBP+2/3 \cdot DBP$.

Further, the step of performing spectrum envelope processing on the first Doppler blood flow signals to obtain a first spectrum envelope curve specifically includes:

continuously acquiring a plurality of columns of first power spectrum densities $S_1(n)$ of the first Doppler blood flow signals to obtain a first integral curve $P_1(n)$ corresponding to each column of the first power spectrum densities $S_1(n)$;

determining a first maximum flow velocity point on the first integral curve $P_1(n)$, and connecting the determined first maximum flow velocity point corresponding to each column to obtain the first spectrum envelope curve.

It should be noted that both of the first and second Doppler blood flow signals are Doppler blood flow signals, and the only difference between them is that they are in different application environments during the calculation. Therefore, the recited "first" and "second" can be uniquely determined according to the current application environment when there is no description on them, which includes but not limited to the description of the Doppler blood flow signal.

As shown in FIG. 5, firstly, the power spectrum density S(n) of a certain column is integrated with the increase of frequency (corresponding to the accumulation of grayscale from low frequency to high frequency in each column of the spectrogram) to form the integral curve P(n) (i.e., a discrete data point curve) of the power spectrum density integral of this column. Secondly, an original point is connected with a last point of the power spectrum density integral to form a straight line. An intersection point (Vcross, P(Vcross)) of the straight line and the integral curve P(n) is also the maximum energy point of the signal. Thirdly, a horizontal ordinate Slowest of a minimum point is searched from S(1) to S(Vcross), a new integral curve P(m) from S(Slowest) to S(2 • Vcross -Slowest) is calculated, and a starting point of P(m) and an end point of P(m) are connected to obtain a new reference straight line. A frequency point corresponding to a maximum positive distance from the reference straight line is the maximum flow velocity point of the signal. At last, the maximum flow velocity points of each column are connected to obtain the envelope curve of the spectrum data, that is, the spectrum envelope curve.

In the present embodiment, frequency characteristics of the power spectrum density integral curve of the ultrasonic Doppler blood flow signals are used to estimate the maximum flow velocity, where the flow velocity is corresponding to the frequency, and the flow velocity is obtained by converting the frequency. The conversion is well known to those skilled in the art, has a small amount of calculation, and has high calculation efficiency, which meets the clinical requirement for real-time and rapid performance of a system.

In an optional embodiment of the present application, the specific step of calculating the initial blood flow parameter and the current blood flow parameter by performing spectrum envelope processing on the second Doppler blood flow signals includes:

> performing the spectrum envelope processing on the second Doppler blood flow signals to obtain a second spectrum envelope curve;
> sequentially identifying each of second pulse cycles of the second spectrum envelope curve to obtain corresponding second pulse cycle curves; and
> extracting the initial blood flow parameter and the current blood flow parameter from initial and final second pulse cycle curves, where the blood flow parameter includes a maximum blood flow velocity, a velocity time integral, and a heart rate.

The blood pressure value is highly correlated with hemodynamic parameters. In the embodiment of the present application, the maximum blood flow velocity Vmax, the velocity time integral Vti and the heart rate Hr in the blood flow parameter are used as the basis for calculation, and the initial blood flow parameter under an initial state and the current blood flow parameter under a final state are obtained by performing spectrum processing and analysis on the continuously acquired second Doppler blood flow signals.

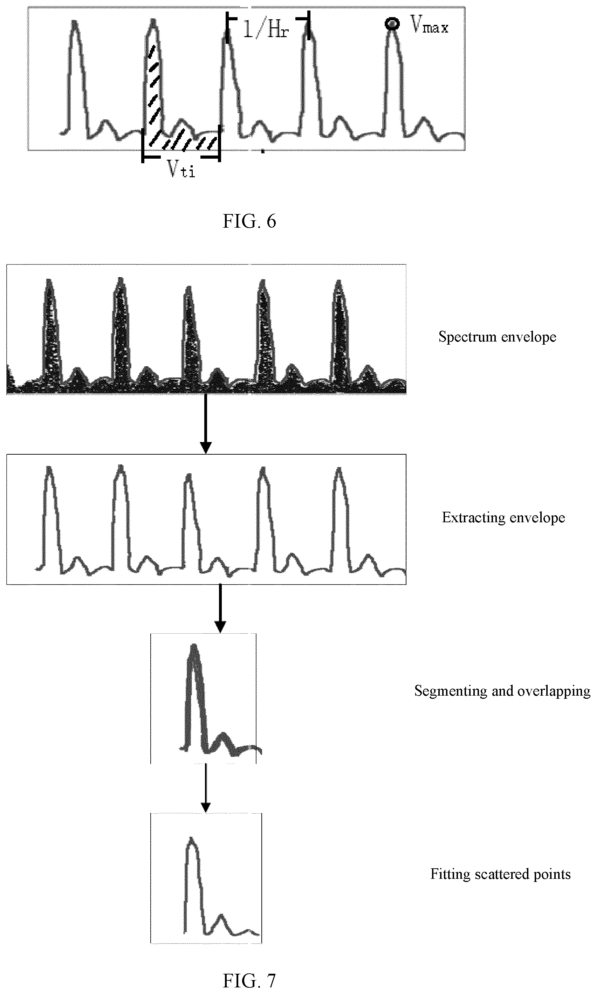

Vmax represents the maximum blood flow velocity, which is inversely proportional to the systolic blood pressure; Vti represents the velocity time integral, that is, a blood volume flowed per beat in unit area, and Vti·S (blood vessel area) represents the blood volume obtained per beat at a measuring point. In the human circulation, the stroke volume per beat flows to the whole body through different blood vessels in a certain proportion, so it is considered that Vti at the measuring point can be substituted for the stroke volume per beat in the calculation process. Hr represents the heart rate, which can be calculated through the pulsation cycle. As shown in FIG. 6, the maximum blood flow velocity Vmax, the velocity time integral Vti, and the heart rate Hr can be determined from the second spectrum envelope curve.

In the present embodiment, the spectrum calculation process of the second Doppler blood flow signals is the same as that of the first Doppler blood flow signals, which is not described in detail again herein.

Generally speaking, if the blood flow parameters are extracted from a single pulse cycle curve, an accidental deviation may occur. Therefore, optionally, the specific step of extracting the initial blood flow parameter and the current blood flow parameter from initial and final second pulse cycle curves includes:

overlapping and fitting a plurality of initial and final second pulse cycle curves, and extracting the initial blood flow parameter and the current blood flow parameter from the fitted initial and final second pulse cycle curves.

In the embodiment of the present application, the specific step of overlapping and fitting a plurality of initial and final second pulse cycle curves, and extracting the initial blood flow parameter and the current blood flow parameter from the fitted initial and final second pulse cycle curves includes:

> identifying and segmenting each of the second pulse cycles of the second spectrum envelope curve to obtain a plurality of second pulse cycle curves;
> overlapping and fitting initial continuous N second pulse cycle curves, and extracting an initial maximum blood flow velocity $Vmax_0$, an initial velocity time integral $Vti_0$, and an initial heart rate $Hr_0$ from the fitted initial curve, where N is a positive integer greater than 1; and
> overlapping and fitting current continuous N second pulse cycle curves, and extracting a current maximum blood flow velocity $Vmax_n$, a current velocity time integral $Vti_n$, and a current heart rate $Hr_n$ from the fitted current curve.

As shown in FIG. 7, in the embodiment of the present application, no matter the spectrum envelope curve or the pulse fluctuation curve, continuous periodic changes will occur, and then each curve is segmented and stored as a plurality of groups of data according to the pulse cycle (also referred to as the cardiac cycle). The number of cardiac cycles is five in the present embodiment. The data of the spectrum envelope curve or the pulse fluctuation curve corresponding to the five cardiac cycles are overlapped. A first cardiac cycle curve is adjusted with the original coordinate as the starting point to obtain a graph, in which five vertical coordinates are corresponding to one horizontal coordinate, and scattered points on the graph are fitted to obtain a new curve, and the new curve is divided into an initial curve and a current curve according to the overlapped object. In the present embodiment, data of the plurality of cardiac cycles are selected for overlapping and superposition, and the overlapped scattered points are fitted to be used for subsequent processes, which can reduce random errors and improve the reliability of the blood pressure measurement result.

Further, the specific step of obtaining the current blood pressure by correcting the standard blood pressure according to the initial blood flow parameter and the current blood flow parameter includes:

> obtaining the current systolic blood pressure $SBP_n$ by correcting the systolic blood pressure $SBP_0$ in the standard blood pressure according to an initial value and a current value of the maximum blood flow velocity and an initial value and a current value of the velocity time integral; and
> obtaining the current diastolic blood pressure $DBP_n$ by correcting the diastolic blood pressure $DBP_0$ in the standard

blood pressure to according to the initial value and the current value of the maximum blood flow velocity and an initial value and a current value of the heart rate.

In the present embodiment, the current systolic blood pressure $SBP_n$ is obtained according to the mathematical Equation:

$$SBP_n=SBP_0+(b \cdot (Vti_n-Vti_0)/Vti_0+a \cdot (Vmax_n-Vmax_0)/Vmax_0) \cdot SBP_0,$$

and

the current diastolic blood pressure $DBP_n$ is obtained according to the mathematical Equation:

$$DBP_n=DBP_0+(x \cdot (Vmax_n-Vmax_0)/Vmax_0+y \cdot (Hr_n-Hr_0)/Hr_0) \cdot DBP_0,$$

in the Equations, a and b respectively represent correction coefficients of relative errors of the maximum blood flow velocity and the velocity time integral in determining the current systolic blood pressure SBPn, x and y respectively represent correction coefficients of relative errors of the maximum blood flow velocity and the heart rate in determining the current diastolic blood pressure DBPn, $SBP_0$ and $DBP_0$ respectively represent the systolic blood pressure and the diastolic blood pressure in the standard blood pressure, SBPn and DBPn respectively represent the systolic blood pressure and the diastolic blood pressure in the current blood pressure, and the correction coefficients are constant.

In order to meet an error accuracy requirement of the measurement result, the optional value ranges of the correction coefficients a, b, x and y are 0.2245~0.2250, 0.0380~0.0385, - 0.01530~-0.01525, and -0.18215~-0.18210, respectively. In a specific embodiment, the specific values of the correction coefficients a, b, x and y are 0.2248, 0.0384, -0.01526, and - 0.18213, respectively.

It can be known that when the mathematical formulas for obtaining the current systolic blood pressure $SBP_n$ and the current diastolic blood pressure $DBP_n$ are stored, and the systolic blood pressure and diastolic blood pressure in the standard blood pressure, the initial value and current value of the maximum blood flow velocity, the initial value and current value of the velocity time integral, the initial value and current value of the heart rate, and each correction coefficient are determined, then the current systolic blood pressure $SBP_n$ and the current diastolic blood pressure $DBP_n$, that is, the current blood pressure can be estimated.

That is, the obtained initial blood flow parameter, the current blood flow parameter, and the standard blood pressure are substituted into a set correction model to obtain the current blood pressure. A mathematical Equation of the correction model includes:

$$SBP_n=SBP_0+(b \cdot (Vti_n -Vti_0)/Vti_0)+a \cdot (Vmax_n -Vmax_0)/Vmax_0) \cdot SBP_0,$$

and

$$DBP_n=DBP_0+(x \cdot (Vmax_n-Vmax_0)/Vmax_0+y \cdot (Hr_n-Hr_0)/Hr_0) \cdot DBP_0.$$

The present application is described in detail below with reference to a specific embodiment.

As shown in FIG. 2, an ultrasonic Doppler sensor is attached to an artery to be measured, and an air pressure cuff is sleeved on the arm to pressurize the artery to be measured through an air pump therein to inflate and block the blood flow. A blood pressure measuring instrument is connected to the ultrasonic Doppler sensor and the air pressure cuff to acquire the Doppler blood flow signals and pressures. At the beginning of the measurement, the systolic blood pressure $SBP_0$ and the diastolic blood pressure $DBP_0$ are measured using a single blood pressure measuring method. When no pressure is applied, the maximum blood flow velocity Vmax, the velocity time integral Vti and the heart rate Hr are calculated by the ultrasonic spectrum envelope.

Firstly, as shown in FIG.3, the air pump in the air pressure cuff is controlled to pressurize, on the one hand, pressure data is read and stored, and on the other hand, ultrasonic signals are acquired periodically and synchronously by the ultrasonic Doppler sensor, that is, the Doppler blood flow signals are acquired. Spectrum calculation and envelope calculation also need to be sequentially performed on the ultrasonic signals acquired in each cycle to acquire a pulse cycle, further a maximum blood flow velocity Vmax is acquired and stored. If the maximum blood flow velocity Vmax acquired in a pulse cycle is less than 0.3m/s, the pressurization is ended and the air is deflated for calculating the standard blood pressure, otherwise, the air pump continues to pressurize.

As shown in FIG. 4, during the calculation of the standard blood pressure, when it is determined that the maximum blood flow velocity Vmax is less than 0.3m/s, the pressure value at this time is the systolic blood pressure $SBP_0$. The plurality of stored maximum blood flow velocity Vmax data are searched, the pressure under the condition that the value of the maximum blood flow velocity Vmax begins to change and the absolute value of the change is approximately 7% is the diastolic blood pressure $DBP_0$.

Thereafter, the ultrasonic signals are continuously collected by the ultrasonic Doppler sensor, and preprocessing and conventional operations, such as digital filtering and FFT calculation, are performed on the collected ultrasonic signal data, and then the spectrum envelope calculation is performed. The initial maximum blood flow velocity $Vmax_0$, the initial velocity time integral $V_{ti0}$, the initial heart rate $Hr_0$, the current maximum blood flow velocity $Vmax_n$, the current velocity time integral $Vti_n$ and the current heart rate $Hr_n$ are analyzed and calculated from continuous spectrum envelope curves, and the envelope curves are overlapped and fitted when necessary.

At last, the calculated initial maximum blood flow velocity $Vmax_0$, initial velocity time integral $Vti_0$, initial heart rate $Hr_0$, current maximum blood flow velocity $Vmax_n$, current velocity time integral $Vti_n$, current heart rate $Hr_n$, the systolic blood pressure $SBP_0$ and the diastolic blood pressure $DBP_0$ are substituted into a set correction model to obtain the current systolic blood pressure $SBP_n$ and the current diastolic blood pressure $DBP_n$. The mathematical Equation of the correction model includes:

$$SBP_n = SBP_0 + (b \cdot (Vti_n - Vti_0)/Vti_0) + a \cdot (Vmax_n - Vmax_0)/Vmax_0) \cdot SBP_0,$$

$$DBP_n = DBP_0 + (x \cdot (Vmax_n - Vmax_0)/Vmax_0 + y \cdot (Hr_n - Hr_0)/Hr_0) \cdot DBP_0,$$

In the Equations, a and b respectively represent correction coefficients of relative errors of the maximum blood flow velocity and the velocity time integral in determining the current systolic blood pressure $SBP_n$, x and y respectively represent correction coefficients of relative errors of the maximum blood flow velocity and the heart rate in determining the current diastolic blood pressure $DBP_n$, $SBP_0$ and $DBP_0$ respectively represent the systolic blood pressure and diastolic blood pressure in the standard blood pressure, $SBP_n$ and $DBP_n$ respectively represent the systolic blood pressure and diastolic blood pressure in the current blood pressure, and the correction coefficients are constant.

As shown in FIG. 2, an embodiment of the present application provides a continuous blood pressure measuring apparatus based on ultrasonic Doppler including:

a pressurization portion (i.e., an air pressure cuff) configured to apply pressure to an artery to be measured;
an ultrasonic Doppler sensor configured to collect Doppler blood flow signals of the artery to be measured;
a blood pressure measuring instrument connected to the ultrasonic Doppler sensor and the pressurization portion, and configured to:

continuously acquire applied pressures during a pressurization operation performed from the beginning to the end on the artery to be measured;
continuously acquire Doppler blood flow signals of the artery to be measured, where the Doppler blood flow signals acquired during the pressurization operation are first Doppler blood flow signals, and the Doppler blood flow signals acquired after the pressurization operation are second Doppler blood flow signals;
obtain a maximum blood flow intensity of each of first pulse cycles according to the first Doppler blood flow signals, and determine a standard blood pressure from all of the acquired pressures according to all of the maximum blood flow intensities and changes thereof; and
calculate an initial blood flow parameter and a current blood flow parameter by performing spectrum envelope processing on the second Doppler blood flow signals, and obtain a current blood pressure by correcting the standard blood pressure according to the initial blood flow parameter and the current blood flow parameter.

In the present embodiment, the ultrasonic Doppler sensor is attached to the artery to be measured, and the air pressure cuff is sleeved on the arm to pressurize the artery to be measured to inflate and block the blood flow. The blood pressure measuring instrument is connected to the ultrasonic Doppler sensor and the air pressure cuff to acquire the Doppler blood flow signals and pressures for single standard blood pressure measurement and non-invasive continuous blood pressure measurement.

In the pressurization operation, the standard blood pressure of the artery to be measured is obtained in a single measurement according to a relationship between the applied pressure and the maximum blood flow intensity in the acquired Doppler blood flow signals. Then, the Doppler blood flow signals of the artery to be measured are continuously acquired in a continuous and non-invasive method without pressurization, and the envelope processing is performed on the continuously acquired Doppler blood flow signals to calculate and obtain the current value and initial value of the blood flow parameter, so as to obtain the current blood pressure by correcting the standard blood pressure according to the obtained current value and initial value of the blood flow parameter, thereby achieving the purpose of non-invasive continuous blood pressure measurement.

As shown in FIG. 8, an embodiment of the present application further provides an electronic device, including:

a processor; and
a memory configured to store computer executable instructions.

The processor is configured to execute the computer executable instructions to implement a continuous blood pressure measuring method based on ultrasonic Doppler as described in any of the above embodiments.

The processor may be a central processing unit (CPU) or other form of processing unit having data processing capability and/or instruction executing capability. The processor may control other components in the electronic device to perform desired functions.

The electronic device may be a terminal, a server or other devices.

The memory may include one or more computer program products, the computer program product may include various forms of computer readable storage media, such as a volatile memory and/or non-volatile memory. For example, the volatile memory may include random access memory (RAM) and/or cache, etc. For example, the non-volatile memory may include read only memory (ROM), hard disk, flash memory, and the like. One or more computer program instructions may be stored on the computer readable storage media, and the processor may execute the program instructions to implement the steps in the continuous blood pressure measuring method and/or other desired functions in each embodiment of the present application described above.

In one example, the continuous blood pressure measuring apparatus may further include: an input apparatus and an output apparatus, and these components are interconnected via a bus system and/or other forms of connection mechanisms (not shown in the drawings).

Of course, for the sake of simplicity, FIG. 8 only shows a part of the components of the continuous blood pressure measuring apparatus related to the present application, components, such as a bus, and the input apparatus/output interface, etc., are omitted. In addition, the continuous blood pressure measuring apparatus may also include any other appropriate components according to specific application conditions.

It should be noted that an embodiment of a continuous blood pressure measuring method based on ultrasonic Doppler, an embodiment of a continuous blood pressure measuring method based on ultrasonic Doppler apparatus, and an embodiment of an electric device provided in the embodiments of the present application belong to the same concept, and each technical feature in the technical solutions described in each embodiment can be arbitrarily combined in the condition that there is no conflict.

It should be understood that the above embodiments are exemplary and are not intended to include all possible implementations included in the claims. Various modifications and changes may be made on the basis of the above embodiments without departing from the scope of the present application. Similarly, each technical feature of the above embodiments may be arbitrarily combined to form other embodiments of the present application that may not be explicitly described. Therefore, the above embodiments only express several implementations of the present application and do not limit the scope of protection of the present application.

**Claims**

1. A continuous blood pressure measuring apparatus based on ultrasonic Doppler, comprising:

an acquisition module, configured to continuously acquire applied pressures during a pressurization operation performed from the beginning to the end on an artery to be measured, and continuously acquire Doppler blood flow signals of the artery to be measured, wherein the Doppler blood flow signals acquired during the pressurization operation are first Doppler blood flow signals, and the Doppler blood flow signals acquired after the pressurization operation are second Doppler blood flow signals;
a determination module, configured to obtain a maximum blood flow intensity of each of first pulse cycles according to the first Doppler blood flow signals, and determine a standard blood pressure from all of the acquired pressures according to all of the maximum blood flow intensities and changes thereof; and

a processing module, configured to perform spectrum envelope processing on the second Doppler blood flow signals to determine an initial blood flow parameter and a current blood flow parameter, and correct the standard blood pressure according to the initial blood flow parameter and the current blood flow parameter to obtain a current blood pressure.

2. The continuous blood pressure measuring apparatus based on ultrasonic Doppler according to claim 1, wherein the determination module is configured to execute the following steps:

performing spectrum envelope processing on the first Doppler blood flow signals to obtain a first spectrum envelope curve;

sequentially identifying each of the first pulse cycles of the first spectrum envelope curve to obtain corresponding first pulse cycle curves; and

extracting a first maximum blood flow velocity from each of the first pulse cycle curves, wherein the first maximum blood flow velocity represents the maximum blood flow intensity.

3. The continuous blood pressure measuring apparatus based on ultrasonic Doppler according to claim 2, further comprises a pressurization portion, wherein the pressurization portion is configured to apply pressures to the artery to be measured, and is specifically configured to, during the pressurization operation performed from the beginning to the end on the artery to be measured, end the pressurization operation if the obtained first maximum blood flow velocity is less than a set blood flow velocity threshold, otherwise, continue the pressurization operation.

4. The continuous blood pressure measuring apparatus based on ultrasonic Doppler according to claim 2, wherein the processing module is configured to execute the following steps:

continuously acquiring a plurality of columns of first power spectrum densities $S_1(n)$ of the first Doppler blood flow signals, and obtaining a first integral curve $P_1(n)$ corresponding to each column of the first power spectrum densities $S_1(n)$; and

determining a first maximum flow velocity point on the first integral curve $P_1(n)$, and connecting the determined first maximum flow velocity point in each column to obtain the first spectrum envelope curve.

5. The continuous blood pressure measuring apparatus based on ultrasonic Doppler according to claim 2 or claim 3, wherein the determination module is configured to execute the following steps:

if the acquired first maximum blood flow velocity is less than a set blood flow velocity threshold, determining a pressure corresponding to the acquired first maximum blood flow velocity as a systolic blood pressure $SBP_0$;

if a change of the acquired first maximum blood flow velocity compared with a first maximum blood flow velocity in a previous first pulse cycle meets a set constraint condition, determining a pressure corresponding to the acquired first maximum blood flow velocity as a diastolic blood pressure $DBP_0$, wherein the constraint condition comprises that a plurality of stored first maximum blood flow velocity data are searched, a pressure when the first maximum blood flow velocity begins to change is the diastolic blood pressure $DBP_0$, and "the first maximum blood flow velocity begins to change" is defined that an absolute value of a change between a current first maximum blood flow velocity and a first maximum blood flow velocity in a previous adjacent pulse cycle is 5%-10%.

6. The continuous blood pressure measuring apparatus based on ultrasonic Doppler according to claim 1, wherein the processing module is configured to execute the following steps:

performing the spectrum envelope processing on the second Doppler blood flow signals to obtain a second spectrum envelope curve;

sequentially identifying each of second pulse cycles of the second spectrum envelope curve to obtain corresponding second pulse cycle curves; and

extracting an initial blood flow parameter and a current blood flow parameter from initial and final second pulse cycle curves, wherein the blood flow parameter comprises a maximum blood flow velocity, a velocity time integral, and a heart rate.

7. The continuous blood pressure measuring apparatus based on ultrasonic Doppler according to claim 6, wherein the processing module is configured to execute the following steps:

overlapping and fitting a plurality of the initial second pulse cycle curves and a plurality of the final second pulse cycle curves, respectively, and extracting the initial blood flow parameter and the current blood flow parameter from the fitted initial and final second pulse cycle curves.

8. The continuous blood pressure measuring apparatus based on ultrasonic Doppler according to claim 6 or claim 7, wherein the processing module is configured to execute the following steps:

correcting a systolic blood pressure $SBP_0$ in the standard blood pressure according to an initial value and a current value of the maximum blood flow velocity and an initial value and a current value of the velocity time integral to obtain a current systolic blood pressure $SBP_n$; and

correcting a diastolic blood pressure $DBP_0$ in the standard blood pressure according to the initial value and the current value of the maximum blood flow velocity and an initial value and a current value of the heart rate to obtain a current diastolic blood pressure $DBP_n$,

wherein the current systolic blood pressure $SBP_n$ is obtained according to a mathematical Equation:

$$SBP_n = SBP_0 + (b \cdot (Vti_n - Vti_0)/Vti_0 + a \cdot (Vmax_n - Vmax_0)/Vmax_0) \cdot SBP_0,$$

and

the current diastolic blood pressure $DBP_n$ is obtained according to a mathematical Equation:

$$DBP_n = DBP_0 + (x \cdot (Vmax_n - Vmax_0)/Vmax_0 + y \cdot (Hr_n - Hr_0)/Hr_0) \cdot DBP_0,$$

wherein in the Equations, a and b respectively represent correction coefficients of relative errors of the maximum blood flow velocity and the velocity time integral in the solution of the current systolic blood pressure $SBP_n$, x and y respectively represent correction coefficients of relative errors of the maximum blood flow velocity and the heart rate in the solution of the current diastolic blood pressure $DBP_n$, $SBP_0$ and $DBP_0$ respectively represent the systolic blood pressure and diastolic blood pressure in the standard blood pressure, and $SBP_n$ and $DBP_n$ respectively represent the systolic blood pressure and diastolic blood pressure in the current blood pressure, and wherein the correction coefficients are constant.

9. The continuous blood pressure measuring apparatus based on ultrasonic Doppler according to any one of claims 1-8, wherein the continuous blood pressure measuring apparatus comprises:

a pressurization portion, configured to apply pressures to the artery to be measured;

an ultrasonic Doppler sensor, configured to collect Doppler blood flow signals of the artery to be measured;

a blood pressure measuring instrument, connected to the ultrasonic Doppler sensor and the pressurization portion and comprising the acquisition module, the determination module, and the processing module.

10. An electronic device, comprising:

a processor; and

a memory configured to store computer executable instructions,

wherein the processor is configured to execute the computer executable instructions to implement a continuous blood pressure measuring method based on ultrasonic Doppler, the continuous blood pressure measuring method comprising the following steps:

continuously acquiring applied pressures during a pressurization operation performed from the beginning to the end on the artery to be measured,

continuously acquiring Doppler blood flow signals of the artery to be measured, wherein the Doppler blood flow signals acquired during the pressurization operation are first Doppler blood flow signals and the Doppler blood flow signals acquired after the pressurization operation are second Doppler blood flow signals;

obtaining a maximum blood flow intensity of each of first pulse cycles according to the first Doppler blood flow signals, and determining a standard blood pressure from all of the acquired pressures according to all of the maximum blood flow intensities and changes thereof; and

calculating an initial blood flow parameter and a current blood flow parameter by performing spectrum envelope processing on the second Doppler blood flow signals, and obtaining a current blood pressure by

14

correcting the standard blood pressure according to the initial blood flow parameter and the current blood flow parameter.

## Patentansprüche

1.  Kontinuierliche Blutdruckmessvorrichtung, die auf Doppler-Ultraschall basiert, umfassend:

    ein Erfassungsmodul, das konfiguriert ist, um kontinuierlich angelegte Drücke während einer Druckbeaufschlagungsoperation zu erfassen, die vom Beginn bis zum Ende an einer zu messenden Arterie durchgeführt wird, und kontinuierlich Doppler-Blutflusssignale der zu messenden Arterie zu erfassen, wobei die während der Druckbeaufschlagungsoperation erfassten Doppler-Blutflusssignale erste Doppler-Blutflusssignale sind, und die nach der Druckbeaufschlagungsoperation erfassten Doppler-Blutflusssignale zweite Doppler-Blutflusssignale sind;
    ein Bestimmungsmodul, das konfiguriert ist, um eine maximale Blutflussintensität von jedem der ersten Pulszyklen gemäß den ersten Doppler-Blutflusssignalen zu erhalten, und einen Standardblutdruck aus allen der erfassten Drücke gemäß allen der maximalen Blutflussintensitäten und deren Veränderungen zu bestimmen; und
    ein Verarbeitungsmodul, das konfiguriert ist, um Spektrumhüllkurvenverarbeitung an den zweiten Doppler-Blutflusssignalen durchzuführen, um einen anfänglichen Blutflussparameter und einen aktuellen Blutflussparameter zu bestimmen, und den Standardblutdruck gemäß dem anfänglichen Blutflussparameter und dem aktuellen Blutflussparameter zu korrigieren, um einen aktuellen Blutdruck zu erhalten.

2.  Kontinuierliche Blutdruckmessvorrichtung, die auf Doppler-Ultraschall basiert, gemäß Anspruch 1, wobei das Bestimmungsmodul konfiguriert ist, um die folgenden Schritte auszuführen:

    Durchführen von Spektrumhüllkurvenverarbeitung an den ersten Doppler-Blutflusssignalen, um eine erste Spektrumhüllkurve zu erhalten;
    sequentielles Identifizieren von jedem der ersten Pulszyklen der ersten Spektrumhüllkurve, um entsprechende erste Pulszykluskurven zu erhalten; und
    Extrahieren einer ersten maximalen Blutflussgeschwindigkeit aus jeder der ersten Pulszykluskurven, wobei die erste maximale Blutflussgeschwindigkeit die maximale Blutflussintensität repräsentiert.

3.  Kontinuierliche Blutdruckmessvorrichtung, die auf Doppler-Ultraschall basiert, gemäß Anspruch 2, die des Weiteren einen Druckbeaufschlagungsabschnitt umfasst, wobei der Druckbeaufschlagungsabschnitt konfiguriert ist, um Drücke an die zu messende Arterie anzulegen, und spezifisch konfiguriert ist, um während der Druckbeaufschlagungsoperation, die vom Beginn bis zum Ende an der zu messenden Arterie durchgeführt wird, die Druckbeaufschlagungsoperation zu beenden, falls die erhaltene erste maximale Blutflussgeschwindigkeit kleiner als eine festgelegte Blutflussgeschwindigkeitsschwelle ist, und ansonsten die Druckbeaufschlagungsoperation fortzusetzen.

4.  Kontinuierliche Blutdruckmessvorrichtung, die auf Doppler-Ultraschall basiert, gemäß Anspruch 2, wobei das Verarbeitungsmodul konfiguriert ist, um die folgenden Schritte auszuführen:

    kontinuierliches Erfassen einer Vielzahl von Spalten von ersten Leistungsspektrumdichten $S_1(n)$ der ersten Doppler-Blutflusssignale, und Erhalten einer ersten Integralkurve $P_1(n)$, die jeder Spalte der ersten Leistungsspektrumdichten $S_1(n)$ entspricht; und
    Bestimmen eines ersten maximalen Flussgeschwindigkeitspunkts auf der ersten Integralkurve $P_1(n)$, und Verbinden des bestimmten ersten maximalen Flussgeschwindigkeitspunkts in jeder Spalte, um die erste Spektrumhüllkurve zu erhalten.

5.  Kontinuierliche Blutdruckmessvorrichtung, die auf Doppler-Ultraschall basiert, gemäß Anspruch 2 oder Anspruch 3, wobei das Bestimmungsmodul konfiguriert ist, um die folgenden Schritte auszuführen:

    falls die erfasste erste maximale Blutflussgeschwindigkeit kleiner als eine festgelegte Blutflussgeschwindigkeitsschwelle ist, Bestimmen eines Drucks, welcher der erfassten ersten maximalen Blutflussgeschwindigkeit entspricht, als systolischen Blutdruck $SBP_0$;
    falls eine Änderung der erfassten ersten maximalen Blutflussgeschwindigkeit, verglichen mit einer ersten maximalen Blutflussgeschwindigkeit in einem früheren ersten Pulszyklus, eine festgelegte Randbedingung erfüllt, Bestimmen eines Drucks, welcher der erfassten ersten maximalen Blutflussgeschwindigkeit entspricht, als diastolischen Blutdruck $DBP_0$, wobei die Randbedingung umfasst, dass eine Vielzahl von gespeicherten ersten maximalen Blutflussgeschwindigkeitsdaten durchsucht wird, wobei ein Druck, wenn die erste maximale Blutflussgeschwindigkeit sich zu verändern beginnt, der diastolische Blutdruck $DBP_0$ ist, und "dass die erste maximale Blutflussgeschwindigkeit sich zu verändern beginnt" so definiert ist, dass ein Absolutwert einer Veränderung zwischen einer aktuellen ersten maximalen Blutflussgeschwindigkeit und einer ersten maximalen Blutflussgeschwindigkeit in einem früheren benachbarten Pulszyklus 5 % bis 10 % beträgt.

6. Kontinuierliche Blutdruckmessvorrichtung, die auf Doppler-Ultraschall basiert, gemäß Anspruch 1, wobei das Verarbeitungsmodul konfiguriert ist, um die folgenden Schritte auszuführen:

   Durchführen der Spektrumhüllkurvenverarbeitung an den zweiten Doppler-Blutflusssignalen, um eine zweite Spektrumhüllkurve zu erhalten;
   sequentielles Identifizieren von jedem der zweiten Pulszyklen der zweiten Spektrumhüllkurve, um entsprechende zweite Pulszykluskurven zu erhalten; und
   Extrahieren eines anfänglichen Blutflussparameters und eines aktuellen Blutflussparameters aus anfänglichen und finalen zweiten Pulszykluskurven, wobei der Blutflussparameter eine maximale Blutflussgeschwindigkeit, ein Geschwindigkeitszeitintegral und eine Herzfrequenz umfasst.

7. Kontinuierliche Blutdruckmessvorrichtung, die auf Doppler-Ultraschall basiert, gemäß Anspruch 6, wobei das Verarbeitungsmodul konfiguriert ist, um die folgenden Schritte auszuführen:
   Überlappen und Anpassen einer Vielzahl von anfänglichen zweiten Pulszykluskurven beziehungsweise einer Vielzahl von finalen zweiten Pulszykluskurven, und Extrahieren des anfänglichen Blutflussparameters und des aktuellen Blutflussparameters aus den angepassten anfänglichen und finalen zweiten Pulszykluskurven.

8. Kontinuierliche Blutdruckmessvorrichtung, die auf Doppler-Ultraschall basiert, gemäß Anspruch 6 oder Anspruch 7, wobei das Verarbeitungsmodul konfiguriert ist, um die folgenden Schritte auszuführen:

   Korrigieren eines systolischen Blutdrucks $SBP_0$ in dem Standardblutdruck gemäß einem anfänglichen Wert und einem aktuellen Wert der maximalen Blutflussgeschwindigkeit und einem anfänglichen Wert und einem aktuellen Wert des Geschwindigkeitszeitintegrals, um einen aktuellen systolischen Blutdruck $SBP_n$ zu erhalten; und
   Korrigieren eines diastolischen Blutdrucks $DBP_0$ in dem Standardblutdruck gemäß dem anfänglichen Wert und dem aktuellen Wert der maximalen Blutflussgeschwindigkeit und einem anfänglichen Wert und einem aktuellen Wert der Herzfrequenz, um einen aktuellen diastolischen Blutdruck $DBP_n$ zu erhalten,
   wobei der aktuelle systolische Blutdruck $SBP_n$ gemäß der folgenden mathematischen Gleichung erhalten wird:

   $$SBP_n = SBP_0 + (b \bullet (Vti_n - Vti_0)/Vti_0 + a \bullet (Vmax_n - Vmax_0)/Vmax_0) \bullet SBP_0$$

   und
   wobei der aktuelle diastolische Blutdruck $DBP_n$ gemäß der folgenden mathematischen Gleichung erhalten wird:

   $$DBP_n = DBP_0 + (x \bullet (Vmax_n - Vmax_0)/Vmax_0 + y \bullet (Hr_n - Hr_0)/Hr_0) \bullet DBP_0,$$

   wobei in den Gleichungen a und b Korrekturkoeffizienten von relativen Fehlern der maximalen Blutflussgeschwindigkeit beziehungsweise des Geschwindigkeitszeitintegrals in der Lösung des aktuellen systolischen Blutdrucks $SBP_n$ repräsentieren, x und y Korrekturkoeffizienten von relativen Fehlern der maximalen Blutflussgeschwindigkeit beziehungsweise der Herzfrequenz in der Lösung des aktuellen diastolischen Blutdrucks $DBP_n$ repräsentieren, $SBP_0$ und $DBP_0$ den systolischen Blutdruck beziehungsweise den diastolischen Blutdruck in dem Standardblutdruck repräsentieren, und $SBP_n$ und $DBP_n$ den systolischen Blutdruck beziehungsweise den diastolischen Blutdruck in dem aktuellen Blutdruck repräsentieren, und wobei die Korrekturkoeffizienten konstant sind.

9. Kontinuierliche Blutdruckmessvorrichtung, die auf Doppler-Ultraschall basiert, gemäß einem der Ansprüche 1 bis 8, wobei die kontinuierliche Blutdruckmessvorrichtung umfasst:

   einen Druckbeaufschlagungsabschnitt, der konfiguriert ist, um Drücke an die zu messende Arterie anzulegen;
   einen Doppler-Ultraschallsensor, der konfiguriert ist, um Doppler-Blutflusssignale der zu messenden Arterie zusammenzutragen;

ein Blutdruckmessinstrument, das mit dem Doppler-Ultraschallsensor und dem Druckbeaufschlagungsabschnitt verbunden ist und das Erfassungsmodul, das Bestimmungsmodul und das Verarbeitungsmodul umfasst.

**10.** Elektronische Vorrichtung, umfassend:

einen Prozessor; und
einen Speicher, der konfiguriert ist, um computerausführbare Anweisungen zu speichern,
wobei der Prozessor konfiguriert ist, um die computerausführbaren Anweisungen auszuführen, um ein kontinuierliches Blutdruckmessverfahren zu implementieren, das auf Doppler-Ultraschall basiert, wobei das kontinuierliche Blutdruckmessverfahren die folgenden Schritte umfasst:

kontinuierliches Erfassen von angelegten Drücken während einer Druckbeaufschlagungsoperation, die vom Beginn bis zum Ende an einer zu messenden Arterie durchgeführt wird, kontinuierliches Erfassen von Doppler-Blutflusssignalen der zu messenden Arterie, wobei die während der Druckbeaufschlagungsoperation erfassten Doppler-Blutflusssignale erste Doppler-Blutflusssignale sind, und die nach der Druckbeaufschlagungsoperation erfassten Doppler-Blutflusssignale zweite Doppler-Blutflusssignale sind;
Erhalten einer maximalen Blutflussintensität von jedem der ersten Pulszyklen gemäß den ersten Doppler-Blutflusssignalen, und Bestimmen eines Standardblutdrucks aus allen der erfassten Drücke gemäß den maximalen Blutflussintensitäten und deren Veränderungen; und
Berechnen eines anfänglichen Blutflussparameters und eines aktuellen Blutflussparameters, indem Spektrumhüllkurvenverarbeitung an den zweiten Doppler-Blutflusssignalen durchgeführt wird, und Erhalten eines aktuellen Blutdrucks, indem der Standardblutdruck gemäß dem anfänglichen Blutflussparameter und dem aktuellen Blutflussparameter korrigiert wird.

## Revendications

**1.** Appareil de mesure continue de la pression artérielle basé sur un écho-Doppler, comprenant :

un module d'acquisition, conçu pour acquérir en continu des pressions appliquées au cours d'une opération de mise sous pression effectuée du début à la fin sur une artère à mesurer, et acquérir en continu des signaux Doppler de flux sanguin de l'artère à mesurer, dans lequel les signaux Doppler de flux sanguin acquis au cours de l'opération de mise sous pression sont des premiers signaux Doppler de flux sanguin, et les signaux Doppler de flux sanguin acquis après l'opération de mise sous pression sont des seconds signaux Doppler de flux sanguin ;
un module de détermination, configuré pour obtenir une intensité maximale de flux sanguin de chacun de premiers cycles d'impulsions en fonction des premiers signaux Doppler de flux sanguin, et déterminer une pression artérielle standard à partir de toutes les pressions acquises en fonction de toutes les intensités maximales de flux sanguin et de variations de celles-ci ; et
un module de traitement, configuré pour effectuer un traitement d'enveloppe spectrale sur les seconds signaux Doppler de flux sanguin afin de déterminer un paramètre de flux sanguin initial et un paramètre de flux sanguin actuel, et corriger la pression artérielle standard en fonction du paramètre de flux sanguin initial et du paramètre de flux sanguin actuel pour obtenir une pression artérielle actuelle.

**2.** Appareil de mesure continue de la pression artérielle basé sur un écho-Doppler selon la revendication 1, dans lequel le module de détermination est configuré pour exécuter les étapes suivantes :

réaliser un traitement d'enveloppe spectrale sur les premiers signaux Doppler de flux sanguin pour obtenir une première courbe d'enveloppe spectrale ;
identifier séquentiellement chacun des premiers cycles d'impulsions de la première courbe d'enveloppe spectrale pour obtenir des premières courbes de cycle d'impulsions correspondantes ; et
extraire une première vitesse maximale de flux sanguin à partir de chaque courbe des premières courbes du cycle d'impulsions, dans lequel la première vitesse maximale de flux sanguin représente l'intensité maximale de flux sanguin.

**3.** Appareil de mesure continue de la pression artérielle basé sur un écho-Doppler selon la revendication 2, qui comprend en outre une partie de mise sous pression, dans lequel la partie de mise sous pression est conçue pour appliquer des pressions à l'artère à mesurer, et est particulièrement conçue pour, au cours de l'opération de mise sous pression effectuée du début à la fin sur l'artère à mesurer, mettre fin à l'opération de mise sous pression si la première vitesse maximale de flux sanguin obtenue est inférieure à un seuil défini de vitesse de flux sanguin, sinon, poursuivre l'opération de mise sous pression.

4. Appareil de mesure continue de la pression artérielle basé sur un écho-Doppler selon la revendication 2, dans lequel le module de traitement est conçu pour exécuter les étapes suivantes :

l'acquisition continue d'une pluralité de colonnes de premières densités de spectre de puissance $S_1(n)$ des premiers signaux Doppler de flux sanguin, et l'obtention d'une première courbe intégrale $P_1(n)$ correspondant à chaque colonne des premières densités de spectre de puissance $S_1(n)$ ; et
la détermination d'un premier point de vitesse maximale de flux sur la première courbe intégrale $P_1(n)$, et la liaison du premier point de vitesse maximale de flux déterminé dans chaque colonne pour obtenir la première courbe d'enveloppe spectrale.

5. Appareil de mesure continue de la pression artérielle basé sur un écho-Doppler selon la revendication 2 ou la revendication 3, dans lequel le module de détermination est configuré pour exécuter les étapes suivantes :

si la première vitesse maximale de flux sanguin acquise est inférieure à un seuil défini de vitesse de flux sanguin, la détermination d'une pression correspondant à la première vitesse maximale de flux sanguin acquise en tant que pression artérielle systolique $SBP_0$ ;
si un changement de la première vitesse maximale de flux sanguin acquise comparée à une première vitesse maximale de flux sanguin dans un premier cycle d'impulsions précédent répond à une condition de contrainte définie, la détermination d'une pression correspondant à la première vitesse maximale de flux sanguin acquise en tant que pression artérielle diastolique $DBP_0$, dans lequel la condition de contrainte comprend le fait qu'une pluralité de premières données stockées de vitesse maximale de flux sanguin est cherchée, une pression à laquelle la première vitesse maximale de flux sanguin commence à changer est la pression artérielle diastolique $DBP_0$, et « la première vitesse maximale de flux sanguin commence à changer » signifie que la valeur absolue d'un changement entre une première vitesse maximale actuelle de flux sanguin et une première vitesse maximale de flux sanguin dans un cycle d'impulsions adjacent précédent est de 5 % à 10 %.

6. Appareil de mesure continue de la pression artérielle basé sur un écho-Doppler selon la revendication 1, dans lequel le module de traitement est conçu pour exécuter les étapes suivantes :

la réalisation du traitement d'enveloppe spectrale sur les seconds signaux Doppler de flux sanguin pour obtenir une seconde courbe d'enveloppe spectrale ;
l'identification séquentielle de chacun des seconds cycles d'impulsions de la seconde courbe d'enveloppe spectrale pour obtenir des secondes courbes de cycle d'impulsions correspondantes ; et
l'extraction d'un paramètre initial de flux sanguin et d'un paramètre actuel de flux sanguin à partir de courbes initiales et finales de second cycle d'impulsions, le paramètre de flux sanguin comprenant une vitesse maximale de flux sanguin, une intégrale temps-vitesse, et une fréquence cardiaque.

7. Appareil de mesure continue de la pression artérielle basé sur un écho-Doppler selon la revendication 6, dans lequel le module de traitement est conçu pour exécuter les étapes suivantes :
le chevauchement et l'ajustement d'une pluralité des secondes courbes initiales de cycle d'impulsions et d'une pluralité des secondes courbes finales de cycle d'impulsions, respectivement, et l'extraction du paramètre initial de flux sanguin et du paramètre actuel de flux sanguin à partir des secondes courbes initiales et finales de cycle d'impulsions.

8. Appareil de mesure continue de la pression artérielle basé sur un écho-Doppler selon la revendication 6 ou revendication 7, dans lequel le module de traitement est configuré pour exécuter les étapes suivantes :

la correction d'une pression artérielle systolique $SBP_0$ dans la pression artérielle standard en fonction d'une valeur initiale et d'une valeur actuelle de la vitesse maximale de flux sanguin et d'une valeur initiale et d'une valeur actuelle de l'intégrale temps-vitesse pour obtenir une pression artérielle systolique actuelle $SBP_n$ ; et
la correction d'une pression artérielle diastolique $DBP_0$ dans la pression artérielle standard en fonction de la valeur initiale et de la valeur actuelle de la vitesse maximale de flux sanguin et d'une valeur initiale et d'une valeur actuelle de la fréquence cardiaque pour obtenir une pression artérielle diastolique actuelle $DBP_n$,
dans lequel la pression artérielle systolique actuelle $SBP_n$ est obtenue selon une équation mathématique :

$$SBP_n = SBP_0 + (b \cdot (Vti_n - Vti_0) / Vti_0 + a \cdot (Vmax, - Vmax_0) / Vmax_0) \cdot SBP_0,$$

et

la pression artérielle diastolique actuelle $DBP_n$ est obtenue selon une équation mathématique :

$$DBP_n = DBP_0 + (x \cdot (Vmax_n - Vmax_0) / Vmax_0 + y \cdot (Hr_n - Hr_0) / Hr_0) \cdot DBP_0,$$

dans lequel, dans les équations, a et b représentent respectivement des coefficients de correction d'erreurs relatives de la vitesse maximale de flux sanguin et de l'intégrale temps-vitesse dans la solution de la pression artérielle systolique actuelle $SBP_n$, x et y représentent respectivement des coefficients de correction d'erreurs relatives de la vitesse maximale de flux sanguin et de la fréquence cardiaque dans la solution de la pression artérielle diastolique actuelle $DBP_n$, $SBP_0$ et $DBP_0$ représentent respectivement la pression artérielle systolique et la pression artérielle diastolique dans la pression artérielle standard, et $SBP_n$ et $DBP_n$ représentent respectivement la pression artérielle systolique et la pression artérielle diastolique dans la pression artérielle actuelle, et dans lequel les coefficients de correction sont constants.

9. Appareil de mesure continue de la pression artérielle basé sur un écho-Doppler selon l'une quelconque des revendications 1 à 8, dans lequel l'appareil de mesure continue de la pression artérielle comprend :

une partie de mise sous pression, conçue pour appliquer des pressions à l'artère à mesurer ;
un capteur d'écho-Doppler, conçu pour collecter des signaux Doppler de flux sanguin de l'artère à mesurer ;
un instrument de mesure de la pression artérielle, connecté au capteur écho-Doppler et à la partie de mise sous pression et comprenant le module d'acquisition, le module de détermination, et le module de traitement.

10. Dispositif électronique, comprenant :

un processeur ; et
une mémoire configurée pour stocker des instructions exécutables par ordinateur,
dans lequel le processeur est configuré pour exécuter les instructions exécutables par ordinateur pour mettre en œuvre un procédé de mesure continue de la pression artérielle basé sur un écho-Doppler, le procédé de mesure continue de la pression artérielle comprenant les étapes suivantes :

l'acquisition continue de pressions appliquées au cours d'une opération de mise sous pression effectuée du début à la fin sur l'artère à mesurer,
l'acquisition continue de signaux Doppler du flux sanguin de l'artère à mesurer, dans lequel les signaux Doppler de flux sanguin acquis pendant l'opération de mise sous pression sont des premiers signaux Doppler de flux sanguin et les signaux Doppler de flux sanguin acquis après l'opération de mise sous pression sont des seconds signaux Doppler de flux sanguin ;
l'obtention d'une intensité maximale de flux sanguin de chacun des premiers cycles d'impulsions en fonction des premiers signaux Doppler de flux sanguin, et la détermination d'une pression artérielle standard à partir de toutes les pressions acquises en fonction de toutes les intensités maximales de flux sanguin et de variations de celles-ci ; et
le calcul d'un paramètre de flux sanguin initial et d'un paramètre de flux sanguin actuel en réalisant un traitement d'enveloppe spectrale sur les seconds signaux Doppler de flux sanguin, et l'obtention d'une pression artérielle actuelle en corrigeant la pression artérielle standard en fonction du paramètre initial de flux sanguin et du paramètre actuel de flux sanguin.

Pressure

| Obtaining a maximum blood flow intensity of each of first pulse cycles according to the first Doppler blood flow signals, and determining a standard blood pressure from all of the acquired pressures according to all of the maximum blood flow intensities and changes thereof |
|---|

First Doppler blood flow signals

Standard blood pressure

| Calculating an initial blood flow parameter and a current blood flow parameter by performing spectrum envelope processing on the second Doppler blood flow signals, and obtaining a current blood pressure by correcting the standard blood pressure according to the initial blood flow parameter and the current blood flow parameter |
|---|

Second Doppler blood flow signals

FIG. 1

Ultrasonic Doppler Sensor

Air Pressure Cuff

Blood Pressure Measuring Instrument

FIG. 2

```
                    ┌─────────────────┐
                    │      Start      │
                    └─────────────────┘
                             │
                             ▼
              ┌──────────────────────────────┐
              │    Pressurizing by air pump   │──────────────────────┐
              └──────────────────────────────┘                       │
                   │                    │                             │
                   ▼                    ▼                             │
         ┌──────────────────────┐  ┌──────────────────────┐          │
         │ Acquiring ultrasonic │  │ Reading pressure data │          │
         │       signals        │  └──────────────────────┘          │
         └──────────────────────┘             │                      │
                   │                           │                      │
                   ▼                           │                      │
         ┌──────────────────────┐              │                      │
         │ Spectrum calculation │              │                      │ N
         └──────────────────────┘              │                      │
                   │                           │                      │
                   ▼                           │                      │
         ┌──────────────────────┐              │                      │
         │ Envelope calculation │              │                      │
         └──────────────────────┘              │                      │
                   │                           │                      │
                   ▼                           │                      │
         ┌──────────────────────┐              │                      │
         │ Acquiring one pulse  │              │                      │
         │        cycle         │              │                      │
         └──────────────────────┘              │                      │
                   │                           │                      │
                   ▼                           │                      │
         ┌──────────────────────┐              │                      │
         │    Acquiring Vmax    │──────────────┤                      │
         └──────────────────────┘              │                      │
                                               ▼                      │
                          ┌──────────────────────────────────┐       │
                          │ Storing pressure value and Vmax   │       │
                          │              value                │       │
                          └──────────────────────────────────┘       │
                                               │                      │
                                               ▼                      │
                                    ◇─────────────────◇               │
                                   ⟨  Vmax<set value   ⟩──────────────┘
                                    ◇─────────────────◇
                                               │ Y
                                               ▼
                          ┌──────────────────────────────────┐
                          │ Ending pressurization and         │
                          │         deflating air             │
                          └──────────────────────────────────┘
                                               │
                                               ▼
                          ┌──────────────────────────────────┐
                          │ Calculating standard blood        │
                          │            pressure               │
                          └──────────────────────────────────┘
                                               │
                                               ▼
                                    ┌────────────────────┐
                                    │        End         │
                                    └────────────────────┘
```

FIG. 3

Vmax (m/s)

1. 3          Diastolic Blood Pressure DBP

1. 0

0. 5                      Mean Arterial Pressure MAP

0. 3                                    Systolic Blood Pressure SBP

0          100          200

Pressure Value mmHg

FIG. 4

Start

Inputting Column data S(n)

Integrating the power spectrum density over frequency to obtain P(n)

Connecting an original point with a last point of the power spectrum density integral by a straight line to obtain an intersection point (Vcross, P(Vcross)) between the straight line and P(n)

Determining a vertical coordinate lowest of a minimum value from S(1) to S(Vcross)

Determining a new intergral curve P(m) from S(Slowest) to S(2*Vcross-Slowest)

Connecting a starting point and an end point of P(m) to form a straight line, and determining maximum positive and negative distances from the intergral curve P(m) to the straight line

Determining a frequency point corresponding to the maximum positive distance as the maximum flow velocity point

End

FIG. 5

FIG. 6

Spectrum envelope

Extracting envelope

Segmenting and overlapping

Fitting scattered points

FIG. 7

Electronic device

Processor — Memory

FIG. 8

Continuous Blood Pressure
Measuring Apparatus Based
On Ultrasonic Doppler

Acquisition Module

Determination Module

Processing Module

FIG. 9

**EP 4 534 017 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210568275X
- US 2015230774 A1